# EUROPÄISCHE PATENTANMELDUNG

(11) **EP 0 959 094 A1**
(43) Veröffentlichungstag der Anmeldung: **24.11.1999**
(21) Anmeldenummer: 99109058.0
(22) Anmeldetag: 07.05.1999
(51) Int. Cl.: C08G 73/10, C08G 73/02, C08G 69/10, A61K 7/48, A61K 7/06, C11D 3/37

(54) **Kondensationsprodukte enthaltend polyethermodifizierte Monoester und/oder -amide Alpha ,Beta-ungesättigter Dicarbonsäuren, ihre Herstellung und Verwendung**

(30) Priorität: 20.05.1998 DE 19822604
(71) Anmelder: Th. Goldschmidt AG, 45127 Essen (DE)
(72) Erfinder: Duetsch, Michael Dr., 45239 Essen (DE); Grüning, Burghard Dr., 45134 Essen (DE); Simpelkamp, Jörg Dr., 45130 Essen (DE); Weitemeyer, Christian, 45134 Essen (DE)

(57) **Zusammenfassung**

Diese Erfindung beschreibt Kondensationsprodukte aus polyethermodifizierten und gegebenenfalls hydrophob modifizierten Monoestern und/oder -amiden α,β-ungesättigter Dicarbonsäuren und Ammoniak, ihre Herstellung und Verwendung.

Die Kondensationsprodukte enthalten über Ester- und/oder Amidbindungen gebundene Polyoxyalkylenketten, hergestellt aus Monoestern und/oder Monoamiden monoethylenisch ungesättigter Dicarbonsäuren und Ammoniak oder hergestellt aus den Ammoniumsalzen der genannten Säuren, sowie deren Gemischen daraus, gegebenenfalls in Gegenwart cokondensierbarer Verbindungen.

## Beschreibung

Diese Erfindung beschreibt Kondensationsprodukte aus polyethermodifizierten und gegebenenfalls hydrophob modifizierten Monoestern und/oder -amiden ,β-ungesättigter Dicarbonsäuren und Ammoniak, ihre Herstellung und Verwendung.

Polyaminosäurederivate, insbesondere Polyasparaginsäure, haben in jüngster Zeit aufgrund ihrer Eigenschaften, insbesondere ihrer biologischen Abbaubarkeit und Naturnähe, besondere Aufmerksamkeit gefunden. Es werden unter anderem Anwendungen als biologisch abbaubare Komplexierungsmittel, Enthärter und Waschmittel-Builder vorgeschlagen. Polyasparaginsäure wird i.A. durch alkalische Hydrolyse der unmittelbaren Synthesevorstufe Polysuccinimid (PSI, Anhydropolyasparaginsäure), dem cyclischen Imid der Polyasparaginsäure gewonnen. PSI kann beispielsweise nach EP 0 578 449 A, WO 92/14753, EP 0 659 875 A oder DE 44 20 642 A aus Asparaginsäure hergestellt werden oder ist beispielsweise nach DE 36 26 672 A, EP 0 612 784 A, DE 43 00 020 A oder US 5 219 952 A aus Maleinsäurederivaten und Ammoniak zugänglich. Für diese üblichen Polyasparaginsäuren werden unter anderem Anwendungen als Inkrustationsinhibitor, Builder in Waschmitteln, Düngemitteladditiv und Hilfsstoff in der Gerberei vorgeschlagen.

DE 4327494, ER 0578449 und ER 0578450 beschreiben die Herstellung von Polysuccinimid aus Asparaginsäure oder Maleinsäurederivaten in Polyethylenglykolen als Lösungsmittel. Eine Inkorporation des Polyethers in das Produkt findet dabei nicht statt.

Die von verschiedenen Arbeitsgruppen beschriebene Umsetzung von Polysuccinimid mit Aminen führt zu Polyasparaginsäureamiden (Kovacs et al., J. Med. Chem. 1967, 10, 904-7; Neuse, Angew. Makromol. Chem. 1991, 192, 35-50). Die Ringöffnung von Polysuccinimid mit Polyaminen und die nachfolgende alkalische Hydrolyse zur Herstellung von Polyasparaginsäurederivaten für Anwendungen als Superabsorber wird beispielsweise in der WO 95/35337 beschrieben. DE 19525365 beschreibt die Modifiziering von Polysuccinimid mit Aminen und aminofunktionellen Polyethern zur Herstellung von Papierhilfsmitteln.

Kataoka et al. beschreiben in Macromol. Chem. Phys. 1995, 196, 1899-1905, Poly-L-asparaginsäurebenzylester mit endständig angeknüpftem Polyetherblock für pharmazeutische Anwendungen. Die Herstellung dieser enantiomerenreinen Produkte verläuft allerdings sehr aufwendig und unwirtschaftlich über die N-Carboxyanhydride der L-Asparaginsäure.

Von besonderem Interesse unter anderem für Anwendungen als Emulgator, Dispergiermittel und Tensid sind copolymere Polyasparaginsäureester, welche partiell mit langkettigen Fettalkoholen oder deren Derivaten verestert sind. Derartige Verbindungen sind auf Basis von Maleinsäuremonoestern und Ammoniak leicht zugänglich, wie aus der DE 195 45 678 beziehungsweise der EP 96 118 806.7 hervorgeht.

Nachteilig im Eigenschaftsprofil derartiger Polyasparaginsäurederivate mit Carboxylatseitenketten als hydrophiler Komponente ist jedoch die pH-Abhängigkeit des Verhaltens. Durch Änderungen des pH-Wertes treten unerwünschte Veränderungen der Hydrophilie auf, z.B. durch Protonierung von Carboxylatgruppen, was sich in ihren anwendungstechnischen Eigenschaften nachteilig bemerkbar macht, z.B. bezüglich der pH-, Temperatur- und Langzeitstabilität der Zubereitungen beispielsweise im Bereich kosmetischer W/O und O/W-Emulsionen.

Aufgabe der Erfindung war daher die Bereitstellung copolymerer Polyasparaginsäurederivate mit verbesserten Anwendungseigenschaften, welche auch nichtionogene hydrophile Gruppen enthalten.

Die Aufgabe wird erfindungsgemäß gelöst durch Kondensationsprodukte aus polyethermodifizierten sowie gegebenenfalls aus hydrophob modifizierten Monoestern und/oder -amiden ,β-ungesättigter Dicarbonsäuren und Ammoniak.

Die erfindungsgemäßen Kondensationsprodukte lassen sich durch ein Herstellverfahren erhalten, das dadurch gekennzeichnet ist, daß man ein Gemisch von Monoestern und/oder Monoamiden monoethylenisch ungesättigter Dicarbonsäuren mit Ammoniak umsetzt bzw. die Ammoniumsalze dieser Säuren thermisch in das Polymer überführt. Eingesetzt werden können beispielsweise Ester- und Amidderivate der Maleinsäure, Fumarsäure, Itaconsäure, Alkenylbernsteinsäure, Alkylmaleinsäure, Citraconsäure oder deren Ammoniumsalze, vorzugsweise Derivate der Maleinsäure, Fumarsäure oder Itaconsäure, besonders vorzugsweise Maleinsäurederivate der allgemeinen Formeln (I) und (II) 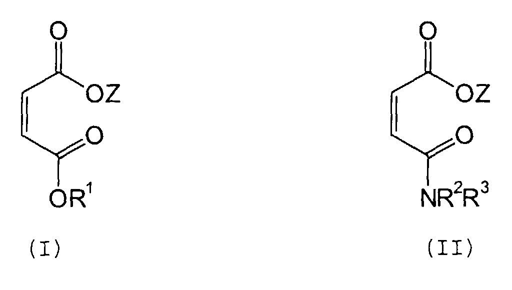 wobei
- R¹: die Bedeutung von R⁴, R⁵, und R⁶ haben kann,
- R²: für gleiche oder verschiedene, geradkettige oder verzweigte, gesättigte oder ungesättigte Alkyl-, Alkenyl- oder Arylreste mit 1 bis 30 C-Atomen, für Hydroxy- oder Aminoalkyl mit 2 bis 30 C-Atomen, jeweils gegebenenfalls Oxa- oder Azasubstituiert, mit 1 bis 6 Hydroxygruppen und/oder 1 bis 6 Aminogruppen, für deren Acylierungsprodukte mit C₁ bis C₂₂ Carbonsäureresten oder für Polyoxyalkylenreste des Typs R¹²X(CₙH₂ₙ₋ₘR¹³ₘO)ₒR¹⁴, worin X Sauerstoff oder Stickstoff, R¹² gleiche oder verschiedene zweiwertige Alkyl-, Alkenyl- oder Arylradikale mit 2 bis 30 C-Atomen, R¹³ Wasserstoff und/oder gleiche oder verschiedene Alkyl-, Alkenyl- oder Arylreste mit 1 bis 30 C-Atomen, R¹⁴ Wasserstoff, gleiche oder verschiedene Alkyl-, Alkenyl-, Aryl- oder Acylreste mit 1 bis 30 C-Atomen bedeutet, n gleich 2 oder 4 ist, m Werte von 0 bis 4 und o Werte von 1 bis 100 annimmt, steht, oder die Bedeutung von R⁵ annimmt, und
- R³: für Wasserstoff oder einen Rest R² steht,
- R⁴: für gleiche oder verschiedene, geradkettige oder verzweigte, gesättigte oder ungesättigte hydroxyfunktionelle Alkyl- oder Alkenylreste mit 1 bis 30 C-Atomen und 1 bis 20 Hydroxygruppen oder deren Acylierungsprodukte mit 1 bis 22 C-Carbonsäureresten, oder für Polyoxyalkylenreste des Typs R¹²X(CₙH₂ₙ₋ₘR¹³ₘO)ₒR¹⁴, worin X Sauerstoff oder der Rest -NH-, R¹² gleiche oder verschiedene zweiwertige Alkyl-, Alkenyl- oder Arylreste mit 2 bis 30 C-Atomen, R¹³ Wasserstoff und/oder gleiche oder verschiedene Alkyl-, Alkenyl- oder Arylreste mit 1 bis 30 C-Atomen, R¹⁴ Wasserstoff, gleiche oder verschiedene Alkyl-, Alkenyl- oder Arylreste mit 1 bis 30 C-Atomen bedeutet, n gleich 2 oder 4 ist, m Werte von 0 bis 4 und o Werte von 1 bis 100 annimmt, steht,
- R⁵: für gleiche oder verschiedene, geradkettige oder verzweigte, gesättigte oder ungesättigte Alkyl- oder Alkenylreste R⁷ mit 6 bis 30 C-Atomen oder für Radikale der Struktur -Y-R⁷, wobei Y die Bedeutung von Polyoxyalkylenresten des Typs (CₙH₂ₙ₋ₘR¹³ₘO)ₒ annimmt, worin R¹³ Wasserstoff und/oder gleiche oder verschiedene Alkyl-, Alkenyl- oder Arylreste mit 1 bis 30 C-Atomen, n gleich 2 oder 4 ist, m Werte von 0 bis 4 und o Werte von 1 bis 100 annimmt, steht, und
- R⁶: für gleiche oder verschiedene, geradkettige oder verzweigte, gesättigte oder ungesättigte Alkyl- oder Alkenylreste mit 1 bis 5 C-Atomen steht,
- z: für einen oder mehrere Reste aus der Gruppe der Alkali-, Erdalkalimetalle, Wasserstoff oder Ammonium, [NR⁸R⁹R¹⁰R¹¹]+ , worin R⁸ bis R¹¹ jeweils unabhängig voneinander für Wasserstoff, gleiche oder verschiedene, geradkettige oder verzweigte, gesättigte oder ungesättigte Alkylreste, Alkenylreste oder hydroxyfunktionelle Alkylreste mit 1 bis 22 C-Atomen und gegebenenfalls 1 bis 6 Hydroxygruppen steht,
und wenigstens ein Rest R², R³ oder R⁴ eine Polyoxyalkylenkette mit 2 bis 100 Einheiten, welche im Falle von R⁴ nicht wie die genannten Radikale Y mit einem Rest R⁷ verbunden ist, enthält.

Alle gegebenen Angaben zur Zusammensetzung der polymeren Produkte beziehen sich wie üblich auf die mittlere Zusammensetzung der Polymerketten.

Eingesetzt werden Mischungen mit einem Gesamtanteil von 1 bis 100 Gew.-%, bevorzugt 40 bis 100 Gew.-% an Esterkomponenten (I) sowie mit einem Anteil von 0 bis 99 Gew.-%, bevorzugt 0 bis 60 Gew.-% an Amidkomponenten (II). Der Gewichtsanteil der Polyoxyalkylenketten am Reaktionsgemisch beträgt 1 bis 95 Gew.-%, vorzugsweise 20 bis 80 Gew.-%. Die Umsetzung erfordert den Einsatz von 0,5 bis 5 Äquivalenten Ammoniak, vorzugsweise 0,8 bis 2 Äquivalente, gasförmig oder in Lösung.

Erfindungsgemäß eingesetzte Maleinsäurederivate des Typs (I), in welchen R¹ die Bedeutung von R⁴ annimmt, können Ester von hydroxyterminalen Alkylenoxidpolymeren sein z.B. auf Basis von Ethylenoxid, Propylenoxid, Butylenoxid, Styroloxid und/oder langkettigen Olefinoxiden oder Tetrahydrofuran, bevorzugt Ester von Polyethylenglykol und Ethylenoxid/Propylenoxid-Copolymeren. Weiterhin bevorzugte Maleinsäureester dieses Typs sind abgeleitet von Polyalkylenglykolen mit einer Hydroxyfunktion an einem Terminus sowie einer Alkyl- oder Acylfunktion am anderen Kettenende, besonders bevorzugt Anlagerungsprodukte von 2 bis 100 Mol Ethylenoxid und /oder Propylenoxid an mono- oder polyfunktionelle Alkohole, Amine, Aminoalkohole oder Carbonsäuren mit 1 bis 5 C-Atomen wie beispielsweise Methanol, Ethanol, die isomeren Propanole und Butanole, Allylalkohol, Ethylenglykol, Ethanolamin, Propylenglykol, Glycerin, Essigsäure sowie Monoacylierungsprodukte von Polyalkylenglykolen beispielsweise mit Carbonsäuren mit 1 bis 4 C-Atomen.

Vorzugsweise eingesetzte Reste R⁵ sind Alkylreste mit 8 bis 30 C-Atomen, beispielsweise lineare oder verzweigte Decyl-, Dodecyl-, Tetradecyl-, Hexadecyl-, oder Octadecylreste sowie ungesättigte Alkenylreste wie beispielsweise Oleyl.

Vorzugsweise eingesetzte Reste R⁶ sind Alkylreste mit 1 bis 4 C-Atomen, vorzugsweise Methyl, Ethyl, n-Propyl, i-Propyl, n-Butyl, i-Butyl, s-Butyl.

Die Einheiten (II) leiten sich bevorzugt von primären oder sekundären Aminen NR²R³H ab, bei denen R² die o.g. Bedeutung hat, beispielsweise verzweigte oder lineare Octyl-, Decyl-, Dodecyl-, Tetradecyl-, Hexadecyl-, Octadecyl-, Docosylreste, auch ungesättigte und mehrfach ungesättigte Spezies wie beispielsweise Oleyl, und R³ Wasserstoff oder Methyl ist. Weiterhin bevorzugte Amine sind Aminoalkohole, beispielsweise Ethanolamin, aminofunktionalisierte Polyethylenglykole, Diethanolamin, Aminopropanol, deren N-Methylderivate, sowie deren Anlagerungsprodukte von 1-30 Mol Ethylenoxid und/oder Propylenoxid, auch mit alkoxyterminierten, besonders bevorzugt methoxyterminierten, Polyoxyalkylenketten, sowie deren Acylierungsprodukte beispielsweise mit geradkettigen oder verzweigten, gesättigten, oder ungesättigten Carbonsäuren mit 1 bis 30 C-Atomen, besonders bevorzugt mit 8 bis 24 C-Atomen.

Die Reaktion kann mit oder ohne Zusatz von organischen Lösungsmitteln erfolgen. Als Lösungsmittel kommen beispielsweise Alkohole, Ketone, Ester, Oligo- und Poly(alkylen)glykole bzw. - glykolether, Dimethylsulfoxid, Dimethylformamid, N,N-Dimethylacetamid oder N-Methylpyrrolidon sowie deren Gemische und andere in Frage. Bevorzugt eingesetzt werden Alkohole mit 2 bis 4 C-Atomen, davon besonders bevorzugt der kurzkettige Alkohol R⁶OH, sowie Ketone wie beispielsweise Methylisobutylketon oder Methylisoamylketon, oder Alkylester von Carbonsäuren mit 1 bis 4 C-Atomen, wie beispielsweise Essigsäure-sec-Butylester oder Essigsäurepentylester.

Die Reaktion kann optional in Gegenwart von verträglichkeitsfördernden Agenzien durchgeführt werden. Dieses können grenzflächenaktive Verbindungen sein, beispielsweise Anlagerungsprodukte von 1 bis 30 Mol Ethylenoxid und /oder 0 bis 5 Mol Propylenoxid an C₁₂-C₃₀-Fettalkohole und Wollwachsalkohole; Ethylenoxidanlagerungsprodukte von Glycerinmono- und -diestern und Sorbitanmono- und diestern von gesättigten und ungesättigten Fettsäuren mit 6 bis 22 C-Atomen; Anlagerungsprodukte von 2 bis 30 Mol Ethylenoxid und /oder 0 bis 5 Mol Propylenoxid an Fettsäuren mit 12 bis 22 C-Atomen und an Alkylphenole mit 8 bis 15 C-Atomen in der Alkylgruppe; C₁₂-C₁₈-Fettsäurepartialester von Anlagerungsprodukten von 1 bis 30 Mol Ethylenoxid an Glycerin; Anlagerungsprodukte von Ethylenoxid an Fette und Öle, beispielsweise Rizinusöl oder gehärtetes Rizinusöl; Partialester von gesättigten oder ungesättigten C₁₂-C₂₂-Fettsäuren, auch verzweigte oder hydroxysubstituierte, mit Polyolen, beispielsweise Ester von Glycerin, Ethylenglykol, Polyalkylenglykolen, Pentaerythrit, Polyglycerin, Zuckeralkoholen wie Sorbit und Polyglucosiden wie Cellulose; Polysiloxan-Polyalkyl-Polyether-Copolymere und deren Derivate sowie hydrophob modifizierte Polyasparaginsäurederivate, beispielsweise teilveresterte Polyasparaginsäuren, teilveresterte Polyasparaginsäure-co-Glutaminsäuren oder Kondensate aus Maleinsäuremonoestern und Ammoniak, beispielsweise hergestellt nach dem erfindungsgemäßen Verfahren oder nach DE 195 45 678 A, wobei das Herstellverfahren der genannten Polyaminosäurederivate keinen Einfluß auf deren verträglichkeitsvermittelnde Wirkung hat. Gegebenenfalls kann auch ein gewisser Teil der Produktmischung im Reaktor verbleiben und als Lösungsvermittler für eine folgende Umsetzung dienen.

Als verträglichkeits- bzw. löslichkeitsvermittelnde Agenzien können auch kationische Tenside, beispielsweise aus der Gruppe der quarternären Ammoniumverbindungen, guarternisierten Proteinhydrolisate, Alkylamidoamine, quarternären Esterverbindungen, quarternären Siliconöle oder quarternären Zucker- und Polysaccharidderivate, anionische Tenside beispielsweise aus der Gruppe der Sulfate, Sulfonate, Carboxylate sowie Mischungen derselben, beispielsweise Alkylbenzolsulfonate, α-Olefinsulfonate, α-sulfonierte Fettsäureester, Fettsäureglycerinestersulfate, Paraffinsulfonate, Alkylsulfate, Alkylpolyethersulfate, Sulfobernsteinsäurealkylester, Fettsäuresalze (Seifen), Fettsäureester der Polymilchsäure, N-Acylaminosäureester, N-Acyltaurate, Acylisethionate, Ethercarboxylate, Monoalkylphosphate, N-Acylaminosäurederivate wie N-Acylaspartate oder N-Acylglutamate, N-Acylsarcosinate, amphotere oder zwitterionische Tenside wie beispielsweise Alkylbetaine, Alkylamidoalkylbetaine des Typs Cocoamidopropylbetain, Sulfobetaine, Phosphobetaine, Sultaine und Amidosultaine, Imidazoliniumderivate, Amphoglycinate, oder nichtionische Tenside wie beispielsweise oxethylierte Fettalkohole, oxethylierte Alkylphenole, oxethylierte Fettsäureester, oxethylierte Mono-, Di- oder Triglyceride oder Polyalkylenglykolfettsäureester, Zuckerester, z.B. Fettsäureester der Saccharose, Fructose oder des Methylglucosids, Sorbitolfettsäureester und Sorbitanfettsäureester (gegebenenfalls oxethyliert), Alkyl- oder Alkenylpolyglucoside und deren Ethoxylate, Fettsäure-N-alkylpolyhydroxyalkylamide, Polyglycerinester, Fettsäurealkanolamide, langkettige tertiäre Aminoxide oder Phosphinoxide sowie Dialkylsulfoxide enthalten sein.

Vorzugsweise verbleiben die verträglichkeitsfördernden Agenzien im Produkt. Die Umsetzung zum Copolymeren erfolgt in einer bevorzugten Ausführungsweise mit wässrigem oder gasförmigen Ammoniak bei Temperaturen von 0 bis 150°C, vorzugsweise 50-140°C sowie nachfolgendem Ausdestillieren bei 70 bis 240°C, vorzugsweise 110 bis 150°C, unter vermindertem Druck, beispielsweise in Knetapparaturen, Hochviskoreaktoren, Extrudern und Rührreaktoren, gegebenenfalls unter Einsatz scherkraftreicher Rührer wie Mig- oder Intermig-Rührer.

Unter den Reaktionsbedingungen werden gleichzeitig ein Teil der Estergruppen, bevorzugt die von R⁶OH abgeleiteten, hydrolysiert und zusätzlich zu den Polyethergruppen weitere hydrophile Gruppen in Form von Carbonsäure- bzw. Carboxylatgruppen freigesetzt. Durch nachfolgende milde partielle oder vollständige Hydrolyse weiterer Esterfunktionen kann, wenn gewünscht, der Anteil freier Säuregruppen weiter erhöht werden, beispielsweise durch Umsetzung mit Wasser gegebenenfalls in Gegenwart von Säuren oder Basen, oder mit Alkalimetallhydroxiden, ggf. in Gegenwart eines organischen Solvens oder Cosolvens.

Die Ester- und Amidkomponenten können auch Mischungen aus Verbindungen mit unterschiedlichen Resten R², R³, R⁴, R⁵ bzw. R⁶ sein.

Es kann auch Maleinsäureanhydrid mit den entsprechenden Alkoholen und Aminen umgesetzt und in situ ohne Isolierung der Maleinsäuremonoester und -amide nach dem erfindungsgemäßen Verfahren kondensiert werden.

Als Struktur für die erfindungsgemäßen Kondensationsprodukte wird, ohne daß die Erfindung darauf beschränkt wird, eine im wesentlichen von Polyasparaginsäure abgeleitete Polymerstruktur angenommen die Struktureinheiten der allgemeinen Formeln (I) und/oder (II) enthält, wobei die Strukturelemente A gleiche oder verschiedene trifunktionelle Kohlenwasserstoffradikale mit 2 C-Atomen der Struktur (A1) oder (A2) sind,

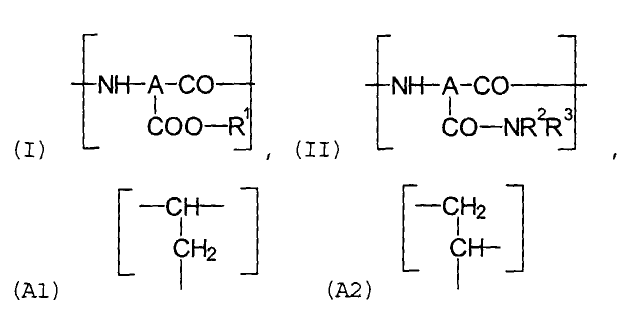

Anstelle der beschriebenen Maleinsäurederivate oder im beliebigen Gemisch mit diesen können auch analoge Derivate der Fumarsäure eingesetzt werden. Weiterhin können erfindungsgemäße Kondensationsprodukte neben Malein- oder Fumarsäurederivaten auch aus den analogen Derivaten der Itaconsäure, Alkenylbernsteinsäure, Alkylmaleinsäure, Citraconsäure hergestellt werden, welche zu 0 bis 100%, vorzugsweise 0 bis 20% in den Reaktionsmischungen enthalten sind.

Durch Zusatz von amino- und carboxyfunktionellen Verbindungen zur Reaktionsmischung können erfindungsgemäß Kondensationsprodukte erhalten werden, in denen die zugesetzten Verbindungen über Amidbindungen gebunden vorliegen. Geeignete Bausteine sind Aminosäuren aus der Gruppe der 20 proteinogenen Aminosäuren, welche als Monomere in allen natürlichen Proteinen enthalten sind, in enantiomerenreiner oder racemischer Form, wie beispielsweise Glutaminsäure, Glutamin, Asparagin, Lysin, Alanin, Glycin, Tyrosin, Tryptophan, Serin und Cystein sowie deren Derivate, oder nicht proteinogenen Aminosäuren mit jeweils einer oder mehreren Amino- bzw. Carboxyfunktionen, wie beispielsweise β-Alanin, ω-Amino-1-alkansäuren. Die Bausteine in einer Menge von 0 bis 20 Gew.-% werden der Ausgangsmischung der Maleinsäurederivate zugesetzt oder zur Modifizierung der Kettenenden nach erfolgter Synthese des Polymeren in üblicher Weise mit diesem umgesetzt, vorzugsweise unter Zusatz polarer Solventien, wie beispielsweise Alkoholen oder Dimethylformamid.

Durch Zusatz von cokondensierbaren Verbindungen zur Reaktionsmischung wie Mono-, Di- oder Polycarbonsäuren wie beispielsweise Maleinsäure, Fumarsäure, Itaconsäure oder gesättigte oder ungesättigte C₈- bis C₂₂-Alkylcarbonsäuren, sowie von hydroxyfunktionellen Carbonsäuren wie beispielsweise Weinsäure, Zitronensäure, Apfelsäure, sowie den Derivaten der genannten cokondensierbaren Säuren, beispielsweise deren Ester, Amide, oder Anhydride kann das Kondensationsprodukt weiter modifiziert werden. Diese Zusätze können in 0 bis 20 Gew.-%, bezogen auf die Ausgangsmischung, enthalten sein.

Die Molekularmassen der Kondensationsprodukte können durch Zusatz von di- und/oder polyfunktionellen Bausteinen, abgeleitet von einer Di- oder Polyhydroxyverbindung, einer Di- oder Polyaminoverbindung oder Aminoalkoholen, mit einem linearen, verzweigten oder cyclischen, gesättigten, ungesättigten oder aromatischen Kohlenwasserstoffgerüst, gegebenenfalls oxo- oder aza-Analogen mit O- oder N-Atomen in der Kette, oder von Polyalkylenglykolen beziehungsweise Ethylenoxid-Propylenoxid-Copolymeren, erhöht werden. Die Einführung der molekulargewichtserhöhenden Gruppen erfolgt durch Zusatz der polyfunktionellen Amino- bzw. Hydroxyverbindungen bzw. deren Umsetzungsprodukten mit Maleinsäureanhydrid zur Reaktionsmischung oder zum gebildeten Polymeren, gegebenenfalls unter Zusatz von sauren oder lewissauren Katalysatoren.

Die resultierenden Polymeren können nachbehandelt werden, beispielsweise durch Behandlung mit Ammoniak, Umesterungskatalysatoren wie beispielsweise lewissauren Titan-(IV)-Verbindungen, mit Aktivkohle oder anderen Adsorbentien, durch Bleichung mit Oxidationsmitteln wie H₂O₂, Cl₂ , O₃, Natriumchlorit, Natriumhypochlorit etc. oder Reduktionsmitteln wie beispielsweise NaBH₄ oder H₂ in Gegenwart von Katalysatoren, oder Anlagerung von Ethylenoxid und/oder Propylenoxid.

Typische Vertreter der erfindungsgemäßen Kondensationsprodukte in der nicht neutralisierten Form haben beispielsweise folgende Zusammensetzung. Die Formel gibt die idealisierte Verknüpfung in α-Position wieder, wobei die Produkte wenigstens teilweise auch in β-Verknüpfung vorliegen können:

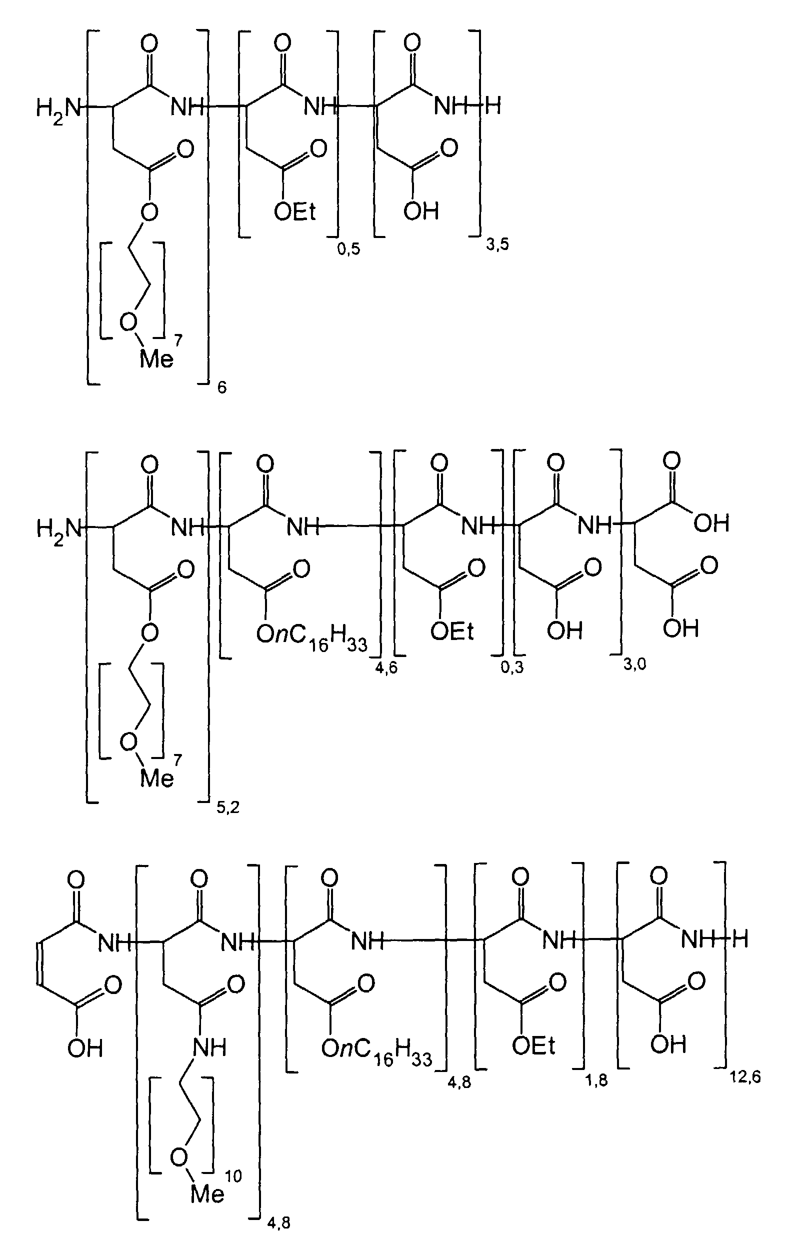

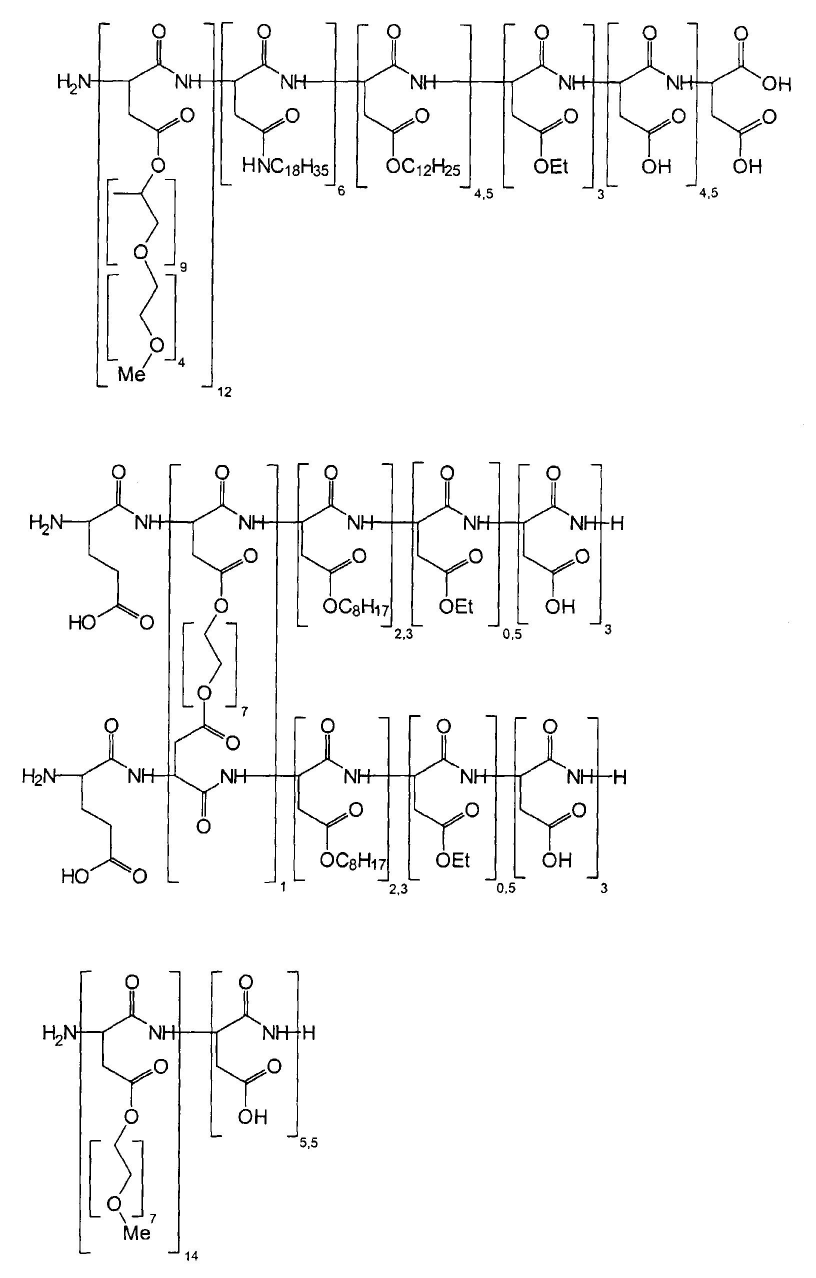

Die erfindungsgemäßen Copolymeren besitzen hervorragende Eigenschaften als Sequestriermittel, als Additive zu Farben und Lacken, als Schaumstabilisatoren, Tenside und Emulgatoren. Insbesondere die pH-, Temperatur- und Langzeitstabilität von O/W- und W/O-Emulsionen wird positiv beeinflusst.

Die erfindungsgemäßen Polymeren können als O/W-Emulgatoren für kosmetische Emulsionen eingesetzt werden, beispielsweise für Lotionen mit einer vergleichsweise niedrigen Viskosität oder Cremes und Salben mit einer hohen Viskosität, für Anwendungen als Hautpflegemittel wie beispielsweise Tagescremes, Nachtcremes, Pflegecremes, Nährcremes, Bodylotions, Salben und dergleichen. Als weitere Hilfs- und Zusatzstoffe können übliche Coemulgatoren, Konsistenzgeber, Ölkörper, Überfettungsmittel, Fette, Wachse, Stabilisatoren, Wirkstoffe, Glycerin, Farb- und Duftstoffe enthalten.

Als Konsistenzgeber können hydrophile Wachse, beispielsweise C₁₂-C₃₀-Fettalkohole, C₁₆-C₂₂-Fettsäuren, Glycerinmono- und -diester und Sorbitanmono- und diester von gesättigten Fettsäuren mit 12 bis 22 C-Atomen eingesetzt werden.

Als weitere Coemulgatoren kommen beispielsweise in Frage: Anlagerungsprodukte von 2 bis 30 Mol Ethylenoxid und /oder 0 bis 5 Mol Propylenoxid an C₁₂-C₃₀-Fettalkohole und Wollwachsalkohole, vorzugsweise lineare, gesättigte C₁₆-C₂₂- Fettalkohole; Ethylenoxidanlagerungsprodukte von Glycerinmono- und -diestern und Sorbitanmono- und diestern von gesättigten und ungesättigten Fettsäuren mit 6 bis 22 C-Atomen; Anlagerungsprodukte von 2 bis 30 Mol Ethylenoxid und/oder 0 bis 5 Mol Propylenoxid an Fettsäuren mit 12 bis 22 C-Atomen und an Alkylphenole mit 8 bis 15 C-Atomen in der Alkylgruppe; C₁₂-C₁₈-Fettsäurepartialester von Anlagerungsprodukten von 1 bis 30 Mol Ethylenoxid an Glycerin; Anlagerungsprodukte von Ethylenoxid an Fette und Öle, beispielsweise Rizinusöl oder gehärtetes Rizinusöl; Partialester von gesättigten oder ungesättigten C₁₂-C₂₂-Fettsäuren, auch verzweigte oder hydroxysubstituierte, mit Polyolen, beispielsweise Ester von Glycerin, Ethylenglykol, Polyalkylenglykolen, Pentaerythrit, Polyglycerin, Zuckeralkoholen wie Sorbit und Polyglucosiden wie Cellulose; Polysiloxan-Polyalkyl-Polyether-Copolymere und deren Derivate sowie hydrophob modifizierte Polyasparaginsäurederivate.

Als Coemulgatoren können auch anionische, kationische, nichtionische, amphotere und/oder zwitterionische Tenside, beispielsweise aus der als verträglichkeitsfördernden Agenzien bezeichneten Gruppe ausgewählt sein.

Es können jeweils beliebige Mischungen der obengenannten Konsistenzgeber und Coemulgatoren eingesetzt werden.

Als Ölkörper kommen beispielsweise Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₂₀-Fettsäuren mit verzweigten Alkoholen, Ester von linearen und/oder verzweigten C₆-C₂₀-Carbonsäuren mit mehrwertigen Alkoholen und/oder Guerbetalkoholen, Triglyceride auf Basis von C₆-C₁₀-Fettsäuren, pflanzliche und tierische Öle und Fette, verzweigte primäre Alkohole, substituierte Cyclohexane, Guerbetcarbonate, Dialkylether und/oder aliphatische beziehungsweise naphthenische Kohlenwasserstoffe in Betracht.

Als Überfettungsmittel können beispielsweise Lanolin und Lecithinderivate sowie deren Ethoxylate, Polyolfettsäureester, Monoglyceride und Fettsäurealkanolamide verwendet werden. Es können Siliconverbindungen wie Polydimethylsiloxane, Cyclodimethicone sowie amino-, fettsäure-, alkohol-, epoxy-, fluor, und/oder alkylmodifizierte Siliconverbindungen sowie Wachse wie beispielsweise Bienenwachs, Paraffinwachse oder Mikrowachse enthalten sein. Die Emulsionen können Verdickungsmittel wie Polyacrylsäurederivate oder kationische Polymere wie beispielsweise kationische Cellulose- oder Stärkederivate, kationische Chitin oder Chitosanderivate, kationische Siliconpolymere, Copolymere von Diallylammoniumsalzen beispielsweise mit Acrylamiden, Polyethylenimin enthalten. Weiterhin können Metallsalze von Fettsäuren, beispielsweise Magnesium-, Aluminium- oder Zinkstearat als Stabilisatoren oder Zinksalze der Ricinolsäure als Geruchshemmer enthalten sein. Es können übliche Sonnenschutzwirkstoffe wie Titandioxid, p-Aminobenzoesäure etc., Duftstoffe, Farbstoffe, biogene Wirkstoffe wie Pflanzenextrakte oder Vitaminkomplexe sowie pharmazeutische Wirkstoffe enthalten sein. Weiterhin können die Emulsionen Perlglanzmittel wie Ethylenglykoldistearat sowie die üblichen Konservierungsmittel wie Parabene, Sorbinsäure, Phenoxyethanol und andere enthalten.

Die erfindungsgemäßen Polyasparaginsäurederivate können auch in W/O-Emulsionen eingesetzt werden, beispielsweise als Emulgatoren und/oder Coemulgatoren für die Herstellung von Hautpflegecremes und -lotionen.

Die erfindungsgemäßen Polyasparaginsäurederivate mit einem naturnahen Polyaminosäurerückgrat sind milde Tenside, welche alleine oder in Kombination mit anionischen, kationischen nichtionischen, zwitterionischen und/oder amphoteren Tensiden eingesetzt werden können. Es sind feste, flüssige oder pastöse Zubereitungen möglich, z.B. Seifenstücke, Waschlotionen, Duschgele, Shampoos.

Die in Kombination mit den erfindungsgemäßen Polyasparaginsäurederivaten einsetzbaren Tenside können beispielsweise anionische Tenside aus der Gruppe der Sulfate, Sulfonate, Carboxylate sowie Mischungen derselben sein. Die anionischen Gruppen können in neutralisierter Form vorliegen, mit kationischen Gegenionen aus der Gruppe der Alkalimetalle, Erdalkalimetalle, Ammonium oder substituiertem Ammonium. Eingesetzt werden beispielsweise Alkylbenzolsulfonate, α-Olefinsulfonate, α-sulfonierte Fettsäureester, Fettsäureglycerinestersulfate, Paraffinsulfonate, Alkylsulfate, Alkylpolyethersulfate, Sulfobernsteinsäurealkylester, Fettsäuresalze (Seifen), Fettsäureester der Polymilchsäure, N-Acylaminosäureester, N-Acyltaurate, Acylisethionate, Ethercarboxylate, Monoalkylphosphate, N-Acylaminosäurederivate wie N-Acylaspartate oder N-Acylglutamate, N-Acylsarcosinate, Polyasparaginsäurederivate und andere.

Die in Kombination mit den erfindungsgemäßen Polyasparaginsäurederivaten einsetzbaren Tenside können beispielsweise amphotere oder zwitterionische Tenside sein, beispielsweise Alkylbetaine, Alkylamidoalkylbetaine des Typs Cocoamidopropylbetain, Sulfobetaine, Phosphobetaine, Sultaine und Amidosultaine, Imidazoliniumderivate, Amphoglycinate und andere.

Die in Kombination mit den erfindungsgemäßen Polyapsaraginsäurederivaten einsetzbaren kationischen Tenside sind beispielsweise aus der Gruppe der quarternären Ammoniumverbindungen, quarternisierten Proteinhydrolysate, Alkylamidoamine, quarternären Esterverbindungen, quarternären Siliconöle oder quarternären Zucker- und Polysaccharidderivate ausgewählt.

Die in Kombination mit den erfindungsgemäßen Polyasparaginsäurederivaten einsetzbaren Tenside können beispielsweise nichtionische Tenside sein, beispielsweise oxethylierte Fettalkohole, oxethylierte Alkylphenole, oxethylierte Fettsäureester, oxethylierte Mono-, Di- oder Triglyceride oder Polyalkylenglykolfettsäureester. Andere nichtionische Tenside können aus der Gruppe der Alkylpolysaccharide, beispielsweise Alkyl- oder Alkenylpolyglucoside, Zuckerester, beispielsweise Fettsäureester der Glucose, Saccharose, Fructose oder des Methylglucosids, Sorbitolfettsäureester und Sorbitanfettsäureester (gegebenenfalls oxethyliert), Polyglycerinester, Fettsäurealkanolamide, N-Acylaminozuckerderivate beispielsweise N-Acylglucamine, langkettige tertiäre Aminoxide oder Phosphinoxide sowie Dialkylsulfoxide stammen

Die in Kombination mit den erfindungsgemäßen Polyasparaginsäurederivaten eingesetzten Tenside können auch beliebige Kombinationen aus zwei oder mehr Tensiden der o.g. Kategorien sein.

Die erfindungsgemäßen Tensidzubereitungen können weitere Hilfs- und Zusatzstoffe enthalten wie beispielsweise Wasser und Lösungsmittel beispielsweise aus der Gruppe der Alkohole und Polyole, Verdickungsmittel, Trübungsmittel, beispielsweise Glykolesterderivate; Moisturizer, Emollients wie tierische und pflanzliche Öle, Carbonsäureester, Lanolin, Bienenwachs, Silicone; polymere Agenzien zur Verbesserung des Hautgefühls, konditionierende, pflegende oder pharmazeutisch wirksame Bestandteile wie beispielsweise kationische oder amphotere Polymere, Proteine und Proteinderivate, Lanolinderivate, Panthothensäure, Betain, Polydimethylsiloxane oder deren Derivate, Sonnenschutzwirkstoffe sowie Lösungsvermittler, Stabilisatoren, Geruchsstoffe, Puffersubstanzen, Konservierungsmittel und/oder Farbstoffe.

Die Polyasparaginsäurederivate enthaltenden Tensidzubereitungen lassen sich vorteilhaft anwenden in z.B. Haarshampoos, Duschbäder, Schaumbadzubereitungen, Hand-, Gesichts und Intimreinigungslotionen, Flüssigseifen, Seifenstücke, Rasiercremes, Handwaschpasten, hautfreundlichen Geschirrspülmitteln, Reinigungsmitteln für glatte Oberflächen sowie in Zahncremes.

Die erfindungsgemäßen Polyasparaginsäurederivate können als Dispergiermittel beispielsweise für Lacke und Farben eingesetzt werden. Dort bewirken sie eine günstige Farbstärkeentwicklung sowie eine Verbesserung der Rub-Out-Eigenschaften.

Die erfindungsgemäßen hydrophob modifizierten Polyasparaginsäurederivate werden dazu vorteilhaft mit dem Stand der Technik entsprechenden Neutralisationsmitteln, insbesondere Aminen neutralisiert. Insbesondere bevorzugt ist hier die Verwendung von Dimethylethanolamin oder 2-Amino-2-methylpropanol. Zur Herstellung wäßriger Pigmentpasten werden 0,1 bis 100 Gew.-%, vorzugsweise 0,5 bis 50 Gew.-%, insbesondere 2 bis 15 Gew.-% bezogen auf das Gewicht der Pigmente verwendet. Die hydrophob modifizierten Polyasparaginsäurederivate können bei der erfindungsgemäßen Verwendung entweder vorab mit den zu dispergierenden Pigmenten vermischt werden oder direkt in dem Dispergiermedium (Wasser, eventuelle Glycolzusätze) vor oder gleichzeitig mit der Zugabe der Pigmente und etwaiger anderer Feststoffe gelöst werden. Die Neutralisation kann dabei vor oder während der Herstellung der Pigmentpasten erfolgen. Bevorzugt werden Polyasparaginsäurezubereitungen eingesetzt, welche bereits partiell oder vollständig neutralisiert wurden.

Die erfindungsgemäßen Polyasparaginsäurederivate können auch in beliebigen Gemischen mit weiteren, dem Stand der Technik entsprechenden Dispergieradditiven, beispielsweise aus der Gruppe der Fettsäureakoxylate, Poly(meth)acrylate, Polyester, Polyether etc., eingesetzt werden.

Als Pigmente können in diesem Zusammenhang beispielsweise anorganische oder organische Pigmente, sowie Ruße genannt werden.

Füllstoffe, die beispielsweise in wäßrigen Lacken dispergiert werden können, sind beispielsweise solche auf Basis von Kaolin, Talkum, anderen Silikaten, Kreide, Glasfasern, Glasperlen oder Metallpulvern. Als anorganische Pigmente seien exemplarisch genannt Titandioxid und Eisenoxide. In Betracht zu ziehende organische Pigmente sind beispielsweise Azopigmente, Metallkomplex-Pigmente, Phthalcyaninpigmente, anthrachinoide Pigmente, polycyclische Pigmente, insbesondere solche der Thioindigo-, Chinacridon-, Dioxazin-, Pyrrolopyrrol-, Naphthalintetracarbonsäure-, Perylen-, Isoamidolin(on)-, Flavanthron-, Pyranthron- oder Isoviolanthron-Reihe.

Als Lacksysteme, in denen die erfindungsgemäßen Pigmentpasten aufgelackt werden können, kommen beliebige wäßrige 1K- oder 2K-Lacke in Betracht. Beispielhaft genannt seien wäßrige 1K-Lacke wie beispielsweise solche auf Basis von Alkyd-, Acrylat-, Epoxid-, Polyvinylacetat-, Polyester- oder Polyurethanharzen oder wäßrige 2K-Lacke, beispielsweise solchen auf Basis von hydroxylgruppenhaltigen Polyacrylat- oder Polyesterharzen mit Melaminharzen oder gegebenenfalls blockierten Polyisocyanatharzen als Vernetzer. In gleicher Weise seien auch Polyepoxidharzsysteme genannt.

Die erfindungsgemäßen Polyasparaginsäurederivate können als komplexbildende Agenzien z.B. in Waschmitteln, als Inkrustationsinhibitoren, als Metalldesaktivatoren in Kunststoffen, als Hilfsstoffe in der Leder- und Textilindustrie oder als wirkungsverstärkende Zusatzstoffe zu Pestiziden oder Insektiziden eingesetzt werden. Hochmolekulare Derivate, vorzugsweise nach Modifizierung mit den obengenannten polyfunktionellen Hydroxy- und und Aminoverbindungen, eignen sich auch als Absorbermaterialien.

### Beispiele:

Die erfindungsgemäße Kondensation modifizierter Monoester und/oder -amide α,β-ungesättigter Dicarbonsäuren erfolgte durch Umsetzung der Edukte (Maleinsäuremonoester, N-substituiertes Maleinsäuremonoamid) in Methylisobutylketon mit 1,0 bis 1,5 Äquivalenten Ammoniakgas und Erhitzen i. Vak. auf 120-140°C für 4 bis 6 h. Die Bestimmung der Veresterungsgrade der Polyasparaginsäureseitenketten erfolgte NMR-spektroskopisch, die Angaben sind in Mol-%.

### Beispiel 1

Die Umsetzung von 1,1 Mol Cetylmaleat und 3,0 Mol des Additionsproduktes aus equimolaren Mengen von Maleinsäureanhydrid und Polyethylenglykol-7-monomethylether mit 5,4 Mol Ammoniak in Methylisobutylketon und thermische Behandlung für 4,5 h bei 120°C und Druckabsenkung auf 20 mbar unter Ausdestillieren ergab ein Kondensationsprodukt mit 23 % Cetylester- und 62% Polyoxyethylenesteranteil.

### Beispiel 2

Die Umsetzung von 0,5 Mol N-Stearylmaleamid, 2,0 Mol Ethylmaleat und 1,5 Mol des Additionsproduktes aus equimolaren Mengen von Maleinsäureanhydrid und Polyethylenglykol-7-monomethylether mit 5,5 Mol Ammoniak in Methylisobutylketon und thermische Behandlung für 5 h bei 120 bis 130°C und Druckabsenkung auf 20 mbar unter Ausdestillieren ergab ein Kondensationsprodukt mit 11% N-Stearylamid- und 31% Polyoxyethylenesteranteil, 8% Ethylester und 50% Säuregruppen.

### Beispiel 3

Die Umsetzung von 1,5 Mol Decylmaleat, 1,0 Mol Ethylmaleat und 1,5 Mol des Additionsproduktes aus equimolaren Mengen von Maleinsäureanhydrid und Polyethylenglykol-12-monomethylether mit 5,5 Mol Ammoniak in n-Decanol und thermische Behandlung für 5 h bei 120°C und Druckabsenkung auf 20 mbar unter Ausdestillieren ergab ein Kondensationsprodukt mit 29 % Decylester, 32% Polyoxyethylenesteranteil, 4 % Ethylester und 35 % Säuregruppen.

### Vergleichsbeispiel 1

Die Kondensation von 1,2 Mol Cetylmaleat und 2,8 Mol Ethylmaleat mit 5,2 Mol Ammoniak in Methylisobutylketon ergab ein Produkt mit 27 % Cetylester- und 6 % Ethylesteranteil.

### Beispiele 4-7

### O/W-Emulsionen mit Polyasparaginsäurederivaten

| | |
|---|---|
| Polyaspartatderivat (25% in Wasser, mit NaOH auf pH 5,5 eingestellt) | 2.0% |
| Glycerin | 3.0% |
| Konservierungsmittel | 0.1% |
| Wasser | 70.4% |
| Glycerinmonostearat (Tegin M, Th. Goldschmidt) Tegosoft® CT (Capryl-Caprintriglycerid, Th. Goldschmidt) | 4.5% |
| | 20.0% |

Die mit Natronlauge auf den in der Tabelle angegebenen pH-Wert eingestellte wäßrige Phase und die Ölkörper/Glycerinmonostearatmischung werden bei 70 °C zusammengegeben, intensiv mit einem Rotor-Stator-Homogenisator bearbeitet (SG/220V, 2 min). Die Emulsion (100 ml) wird zwei Tage bei 20 °C und 28 d bei 45 °C gelagert. Die Wasserseparation der O/W-Emulsionen wurde nach 2 d Lagerung bei 20 °C und nach weiteren 28 d Lagerung bei 45 °C bestimmt.

| Beispiel | Emulgator aus Beispiel | pH-Wert wäßrigen Phase | der Wasserseparation nach 2 d, 20°C (vol.-%) | Wasserseparation nach 28 d, 45°C (vol.-%) |
|---|---|---|---|---|
| 3 | 1 | 5.5 | <0.1 | 5 |
| 4 | 1 | 7.0 | <0.1 | 5 |
| 5 (Vergleich) | Verglbsp.1 | 5.5 | <0.1 | 6 |
| 6 (Vergleich) | Verglbsp.1 | 7.0 | 0.4 | 15 |

Diese Ergebnisse zeigen die verbesserter Emulsionsstabilität in variablen pH-Bereichen bei Verwendung der Produkte, die durch Kondensation von erfindungsgemäß modifizierten Monoestern und gegebenenfalls -amiden α,β-ungesättigter Dicarbonsäuren erhalten werden.

## Patentansprüche

1. Kondensationsprodukte, enthaltend über Ester- und/oder Amidbindungen gebundene Polyoxyalkylenketten, hergestellt aus Monoestern und/oder Monoamiden monoethylenisch ungesättigter Dicarbonsäuren und Ammoniak oder hergestellt aus den Ammoniumsalzen der genannten Säuren, sowie deren Gemischen daraus, gegebenenfalls in Gegenwart cokondensierbarer Verbindungen.

2. Kondensationsprodukte nach Anspruch 1, enthaltend über Ester- und/oder Amidbindungen gebundene gleiche oder verschiedene, geradkettige oder verzweigte, gesättigte oder ungesättigte Alkyl- oder Alkenylreste mit 1 bis 30 C-Atomen.

3. Kondensationsprodukte nach Anspruch 1, enthaltend über Ester- und/oder Amidbindungen gebundene, gleiche oder verschiedene, geradkettige oder verzweigte, gesättigte oder ungesättigte Alkyl- oder Alkenylreste mit 6 bis 30 C-Atomen.

4. Kondensationsprodukte nach einem der Ansprüche 1 bis 3, hergestellt aus Maleinsäurederivaten der allgemeinen Formeln (I) und (II) 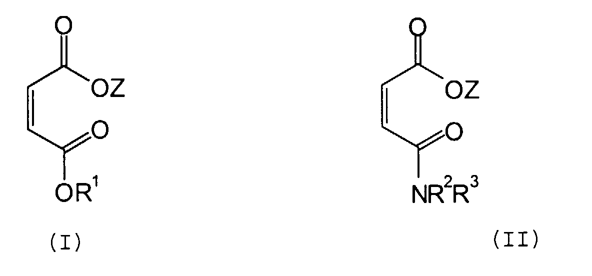 wobei
R¹ die Bedeutung von R⁴, R⁵, und R⁶ haben kann,
R² für gleiche oder verschiedene, geradkettige oder verzweigte, gesättigte oder ungesättigte Alkyl-, Alkenyl- oder Arylreste mit 1 bis 30 C-Atomen, für Hydroxy- oder Aminoalkyl, jeweils gegebenenfalls Oxa- oder Aza-substituiert, mit 1 bis 5 Hydroxygruppen und/oder 1 bis 6 Aminogruppen, für deren Acylierungsprodukte mit C₁ bis C₂₂ Carbonsäureresten oder für Polyoxyalkylenreste des Typs R¹²X(CₙH₂ₙ₋ₘR¹³ₘO)ₒR¹⁴, worin X Sauerstoff oder der Rest -NH-, R¹² gleiche oder verschiedene zweiwertige Alkyl-, Alkenyl- oder Arylradikale mit 2 bis 30 C-Atomen, R¹³ Wasserstoff und/oder gleiche oder verschiedene Alkyl-, Alkenyl- oder Arylreste mit 1 bis 30 C-Atomen, R¹⁴ Wasserstoff, gleiche oder verschiedene Alkyl-, Alkenyl-, Aryl- oder Acylreste mit 1 bis 30 C-Atomen bedeutet, n gleich 2 oder 4 ist, m Werte von 0 bis 4 und o Werte von 1 bis 100 annimmt, steht, oder die Bedeutung von R⁵ annimmt, und
R³ für Wasserstoff oder einen Rest R² steht,
R⁴ für gleiche oder verschiedene, geradkettige oder verzweigte, gesättigte oder ungesättigte hydroxyfunktionelle Alkyl- oder Alkenylreste mit 1 bis 30 C-Atomen und 1 bis 20 Hydroxygruppen oder deren Acylierungsprodukte mit C₁ bis C₂₂ Carbonsäureresten, oder für Polyoxyalkylenreste des Typs R¹²X(CₙH₂ₙ₋ₘR¹³ₘO)ₒR¹⁴, worin X Sauerstoff oder der Rest -NH-, R¹² gleiche oder verschiedene zweiwertige Alkyl-, Alkenyl- oder Arylreste mit 2 bis 30 C-Atomen, R¹³ Wasserstoff und/oder gleiche oder verschiedene Alkyl-, Alkenyl- oder Arylreste mit 1 bis 30 C-Atomen , R¹⁴ Wasserstoff, gleiche oder verschiedene Alkyl- oder Arylreste mit 1 bis 30 C-Atomen, n gleich 2 oder 4 ist, m Werte von 0 bis 4 und o Werte von 1 bis 100 annimmt, steht,
R⁵ für gleiche oder verschiedene, geradkettige oder verzweigte, gesättigte oder ungesättigte Alkyl- oder Alkenylreste R⁷ mit 6 bis 30 C-Atomen oder für Radikale der Struktur -Y-R⁷ , wobei Y die Bedeutung von Polyoxyalkylenresten des TYPS (CₙH₂ₙ₋ₘR¹³ₘO)ₒ annimmt, worin R¹³ Wasserstoff und/oder gleiche oder verschiedene Alkyl-, Alkenyl- oder Arylreste mit 1 bis 30 C-Atomen, n gleich 2 oder 4 ist, m Werte von 0 bis 4 und o Werte von 1 bis 100 annimmt, steht, und
R⁶ für gleiche oder verschiedene, geradkettige oder verzweigte, gesättigte oder ungesättigte Alkyl- oder Alkenylreste mit 1 bis 5 C-Atomen steht,
Z für ein oder mehrere Reste aus der Gruppe der Alkali-, Erdalkalimetalle, Wasserstoff oder Ammonium, [NR⁸R⁹R¹⁰R¹¹]⁺, worin R⁸ bis R¹¹ jeweils unabhängig voneinander für Wasserstoff, gleiche oder verschiedene, geradkettige oder verzweigte, gesättigte oder ungesättigte Alkylreste oder hydroxyfunktionelle Alkylreste mit 1 bis 22 C-Atomen und gegebenenfalls 1 bis 6 Hydroxygruppen steht,
und wenigstens ein Rest R², R³ oder R⁴ eine Polyoxyalkylenkette mit 2 bis 100 Einheiten, welche im Falle von R⁴ nicht wie die genannten Radikale Y mit einem Rest R⁷ verbunden ist, enthält.

5. Kondensationsprodukte nach einem der Ansprüche 1 bis 4, erhältlich durch Umsetzung der Monoester und Monoamide, der Fumarsäure, Itaconsäure, Alkenylbernsteinsäure, Alkylmaleinsäure und/oder Citraconsäure alleine oder im Gemisch mit den Maleinsäurederivaten.

6. Kondensationsprodukte nach Anspruch 1 bis 4, erhältlich durch Umsetzung von Maleinsäuremonoestern von hydroxyfunktionellen Polyoxyalkylenen und Maleinsäuremonoestern von Alkoholen des Typs R⁵OH und/oder R⁶OH und/oder Maleinsäureamiden, abgeleitet von Aminen des Typs NR²R³H, wobei R², R³, R⁵ und R⁶ die obengenannte Bedeutung haben.

7. Kondensationsprodukte nach Anspruch 3, erhältlich durch Umsetzung von Maleinsäuremonoamiden abgeleitet von aminofunktionellen Polyoxyalkylenen und Maleinsäuremonoestern von Alkoholen des Typs R⁵OH und/oder R⁶OH und/oder Maleinsäureamiden, abgeleitet von Aminen des Typs NR²R³H, wobei R², R³, R⁵ und R⁶ die obengenannte Bedeutung haben.

8. Kondensationsprodukte nach Anspruch 1 bis 7, hergestellt in Gegenwart von bis zu 20 Gew.-% cokondensierbaren Verbindungen aus der Gruppe der proteinogenen oder nichtproteinogenen Aminosäuren, mono-, di- oder polyfunktionellen Carbonsäuren, insbesondere Maleinsäure, Fumarsäure, Itaconsäure, Alkenylbernsteinsäure, Citraconsäure, hydroxyfunktionellen Carbonsäuren, sowie deren Ester, Amide, Anhydride oder deren Salze.

9. Kondensationsprodukte nach Anspruch 1 bis 8, welche in Gegenwart von molekularmasseerhöhenden Agenzien aus der Gruppe der Di- oder Polyhdroxyverbindungen, Di- oder Polyaminoverbindungen, oder Aminoalkohole oder Mischungen daraus, mit einem linearen, verzweigten oder cyclischen, gesättigten, ungesättigten oder aromatischen Kohlenwasserstoffgerüst, gegebenenfalls oxo- oder azasubstituiert mit O- oder N-Atomen in der Kette, oder deren Umsetzungsprodukten mit Maleinsäureanhydrid, hergestellt werden oder nach der Herstellung mit diesen modifiziert werden.

10. Kosmetische W/O- oder O/W-Emulsionen, enthaltend Kondensationsprodukte gemäß den Ansprüchen 1 bis 9.

11. Kosmetische Emulsionen nach Anspruch 10, dadurch gekennzeichnet, daß der nichtwäßrige Anteil 5 bis 99 Gew.-% an Ölkörpern aus der Gruppe der Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₂₀-Fettsäuren mit verzweigten Alkoholen, Ester von linearen und/oder verzweigten C₆-C₂₀-Carbonsäuren mit mehrwertigen Alkoholen und/oder Guerbetalkoholen, Triglyceride auf Basis von C₆-C₁₀-Fettsäuren, pflanzliche und tierische Öle und Fette, verzweigte primäre Alkohole, substituierte Cyclohexane, Guerbetcarbonate, Dialkylether und/oder aliphatische bzw. naphthenische Kohlenwasserstoffe enthält.

12. Kosmetische O/W-Emulsionen nach Anspruch 10 und 11, dadurch gekennzeichnet, daß hydrophile Wachse aus der Gruppe der C₁₂-C₃₀-Fettalkohole, Wollwachsalkohole, C₁₆-C₂₂-Fettsäuren, Glycerinmono- und -diester und Sorbitanmono- und diester von gesättigten Fettsäuren mit 12 bis 22 C-Atomen enthalten sind.

13. Kosmetische Emulsionen nach Anspruch 10 bis 12, dadurch gekennzeichnet, daß ein oder mehrere Coemulgatoren aus der Gruppe der Anlagerungsprodukte von Ethylenoxid oder Ethylenoxid und Propylenoxid an C₁₂-C₃₀-Fettalkohole und Wollwachsalkohole, der Ethylenoxidanlagerungsprodukte von Glycerinmono- und -diestern und Sorbitanmono- und diestern von gesättigten und ungesättigten Fettsäuren mit 6 bis 22 C-Atomen, der Anlagerungsprodukte von Ethylenoxid und /oder Propylenoxid an Fettsäuren mit 12 bis 22 C-Atomen und an Alkylphenole mit 8 bis 15 C-Atomen in der Alkylgruppe, der C₁₂-C₁₈-Fettsäuremono- und -diester von Anlagerungsprodukten von Ethylenoxid an Glycerin, der Anlagerungsprodukte von Ethylenoxid an Fette und Öle, der Polyolester von gesättigten oder ungesättigten C₁₂-C₂₂-Fettsäuren, auch verzweigte oder hydroxysubstituierte, Polysiloxan-Polyalkyl-Polyether-Copolymere und deren Derivate, der anionischen Tenside, kationischen Tenside, nichtionischen Tenside sowie zwitterionischen oder amphoteren Tenside enthalten sind.

14. Verwendung der Emulsionen nach Anspruch 10 bis 13 als Hautpflegemittel, Tagescremes, Nachtcremes, Pflegecremes, Nährcremes, Bodylotions, pharmazeutische Salben und Lotionen, Aftershavelotionen, Sonnenschutzmittel.

15. Tensidische Zubereitungen für Reinigungsmittel und/oder kosmetische Mittel, enthaltend Kondensationsprodukte nach Anspruch 1 bis 9, gegebenenfalls enthaltend ein oder mehrere weitere Tenside aus der Gruppe der anionischen, kationische, nichtionischen, amphoteren und Zwitter-ionischen Tenside sowie deren Mischungen daraus neben üblichen Hilfs- und und Zusatzstoffen.

16. Verwendung der Tensidzulbereitungen nach Anspruch 15 für Shampoos, Waschlotionen und Reinigungsmittel für Gesicht, Haar, Haut und Intimbereich, Rasiercreme und -lotionen, Flüssigseifen, Geschirrspülmittel, Reinigungsmittel für glatte Oberflächen, Seifenstücke, Schaumbäder, Duschgele sowie in Zahncremes und/oder Mundspülungen.

17. Verwendung der Copolymeren gemäß den Ansprüchen 1 bis 9 als Waschmittelhilfsstoff, Komplexagens für mehrwertige Kationen, Dispergierhilfsmittel für Lacke und Farben, Inkrustationsinhibitor, Absorbermaterialien, Metalldesaktivatoren in Kunststoffen, als Hilfsstoffe in der Leder und Textilindustrie oder als wirkungsverstärkende Zusatzstoffe zu Pestiziden oder Insektiziden.
